# EUROPEAN PATENT APPLICATION

(11) **EP 4 458 943 A1**
(43) Date of publication of application: **06.11.2024**
(21) Application number: 23171290.2
(22) Date of filing: 03.05.2023
(51) Int. Cl.: C12M 1/33, C12N 5/00

(54) **CELL DISSOCIATION DEVICE AND METHOD**

(71) Applicant: Bayer AG, 51373 Leverkusen (DE)
(72) Inventor: Schwan, Peter, 51373 Leverkusen (DE); Poggel, Martin, 50733 Köln (DE); Tscheschke, Bernd, 51061 Köln (DE); Baas, Markus, 50827 Köln (DE); Sousa, Marcos, 50679 Köln (DE)
(74) Representative: BIP Patents

(57) **Abstract**

What is described herein relates to a method and a device for dissociating cell agglomerations in the range of between >30 µm to 300 µm after harvest into single cells or cell oligomers in the range of 8-30 µm comprising the steps of
• providing cell agglomerations in the range of between >30 µm to 300 µm
• passing said cell agglomerations in the range of between >30 µm to 300 µm through a recirculation loop
• bringing said cell agglomerations in the range of between >30 µm to 300 µm into contact with a shear component exerting a maximal shear in the range of between 2 Pa and 500 Pa
thereby dissociating the cell agglomerations in the range of between >30 µm to 300 µm into single cells or cell oligomers in the range of 8-30 µm.

## Description

An established way of culturing various cell types such as induced pluripotent stem cells, embryoid bodies and embryonic stem cells is suspension culture, where single cells or small aggregates of cells grow and multiply in a growth medium. Dissociation of the cell aggregates is often necessary in order to e.g. reduce aggregate size, reach a uniform aggregate size, achieve a single cell suspension for cell counting, reinoculation or further processing. In general enzymatic or mechanical means or a combination of both can be used for dissociation. With both approaches minimizing cell damage has to be balanced out with dissociation efficiency. If the cells are to be employed in cell therapy applications mechanical dissociation means are preferred in order to avoid any residues of enzymatic dissociation components in the final cell therapy product. However, existing mechanical cell dissociation technologies often fail to deliver a consistent cell quality. One reason being that the dissociation is carried out manually - e.g. via pipetting up-and down - and differences between the manual handlers exist. Another cause for varying cell quality is that if means such sonication are used the mechanical forces negatively impact cell viability. Hence a need exists for a cell dissociation which delivers consistent cell quality.

This need is met by a method for dissociating cell agglomerations in the range of between >30 µm to 300 µm after harvest into single cells or cell oligomers in the range of 8-30 µm comprising the steps of
- providing cell agglomerations in the range of between >30 µm to 300 µm
- passing said cell agglomerations in the range of between >30 µm to 300 µm through a recirculation loop
- bringing said cell agglomerations in the range of between >30 µm to 300 µm into contact with a shear component (2) exerting a maximal shear in the range of between 2 Pa and 500 Pa
thereby dissociating the cell agglomerations in the range of between >30 µm to 300 µm into single cells or cell oligomers in the range of 8-30 µm.

### SUMMARY

In a first aspect what is described herein relates to a method for dissociating cell agglomerations in the range of between >30 µm to 300 µm after harvest into single cells or cell oligomers in the range of 8-30 µm comprising the steps of
- providing cell agglomerations in the range of between >30 µm to 300 µm
- passing said cell agglomerations in the range of between >30 µm to 300 µm through a recirculation loop
- bringing said cell agglomerations in the range of between >30 µm to 300 µm into contact with a shear component (2) exerting a maximal shear in the range of between 2 Pa and 500 Pa thereby dissociating the cell agglomerations in the range of between >30 µm to 300 µm into single cells or cell oligomers in the range of 8-30 µm

In a second aspect what is described herein relates to cell dissociation device (1) for dissociating cell agglomerations in the range of between >30 µm to 300 µm into single cells or cell oligomers in the range of 8-30µm comprising
- at least one shear component (2) exerting a maximal shear in the range of between 2 Pa and 500 Pa
- at least one recirculation loop (3) with a volume in the range of between 200 ml and 1000 ml
- at least one recirculation component (4)
- at least one inlet (5)
- and at least one outlet (6).

### DESCRIPTION OF THE FIGURE

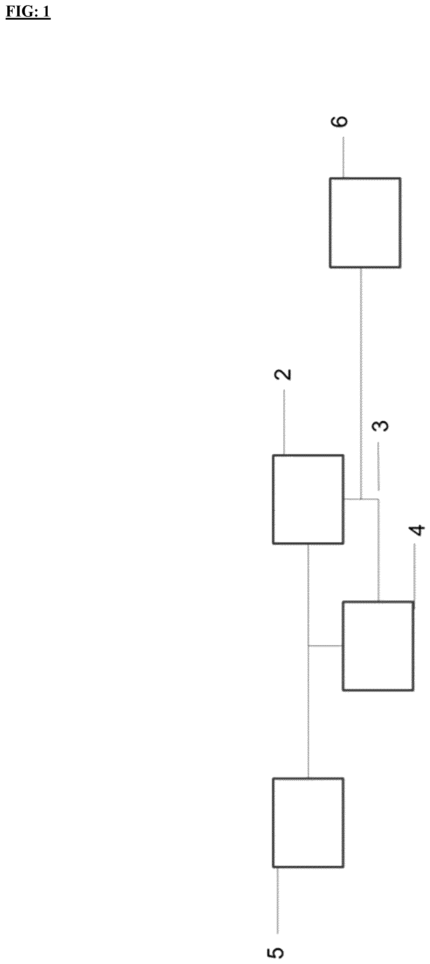

Fig. 1 depicts an exemplary cell dissociation device (1) for dissociating cell agglomerations in the range of between >30 µm to 300 µm into single cells or cell oligomers in the range of 8-30µm comprising a shear component (2) exerting a maximal shear in the range of between 2 Pa and 500 Pa, a recirculation loop (3) with a volume in the range of between 200 ml and 1000 ml, a recirculation component (4), an inlet (5) and an outlet (6).

### DETAILED DESCRIPTION

In a first aspect what is described herein relates to a method for dissociating cell agglomerations in the range of between >30 µm to 300 µm after harvest into single cells or cell oligomers in the range of 8-30 µm comprising the steps of
- providing cell agglomerations in the range of between >30 µm to 300 µm
- passing said cell agglomerations in the range of between >30 µm to 300 µm through a recirculation loop
- bringing said cell agglomerations in the range of between >30 µm to 300 µm into contact with a shear component (2) exerting a maximal shear in the range of between 2 Pa and 500 Pa
thereby dissociating the cell agglomerations in the range of between >30 µm to 300 µm into single cells or cell oligomers in the range of 8-30 µm.

As used herein the term shear component (2) refers to an active device or a passive structure that exerts a maximal shear in the range of between 2 Pa and 500 Pa on the cells to be dissociated while limiting an impact on cell viability. In order to determine whether a given device is suitable as shear component in a given setting methods known in the art can be employed. For example Villiger et al. 2015 (Villiger et al AIChEJ 61-2015) describe how the shear can be assessed using CFD simulations in case of contract nozzles which at certain flow rates and diameters exert a shear stress within the required range of between 2 Pa and 500 Pa (cf. Table 1 of Villiger et al. 2015) also shear stress sensitive Poly(methyl methacrylate (PMMA) particles were developed. Villiger et al. 2018 (Villiger et al . 2018 Biochemical Engineering Journal Volume 131, 15 March 2018, Pages 84-94) describe how shear was CFD modeled for different bioreactor systems exerting between 1 and 20 Pa shear (cf. Fig. 2c of Villiger et al. 2018) and these CFD model calculations were compared to measured shear values obtained in experiments employing the PMMA particles thereby correlating the size of the PMMA aggregates to the corresponding shear. It was found that the predicted values of maximum stress correlated well with those calculated by CFD (Villiger et al. 2018) hence both methods can be employed to determine whether a given device can be used as shear component exerting a shear in the range of between 2-500 Pa. 3. In 2022 Srom et al. verified that the PMMA method can be used to determine the shear in a 250ml ambr250^{®} bioreactor system (Srom et al Biochemical Engineering Journal 177 (2022). Hence in general a person skilled in the art can employ various methods especially CFD simulation and the PMMA method to determine the shear exerted by a given device in a given setting. If one method has to be chosen e.g. to exactly compare different system the PMMA method is to be used.

The maximal shear exerted by the least one shear component (2) is in the range of between 2 Pa and 500 Pa, preferably in the range of between 2 Pa and 100 Pa, more preferably in the range between 2 Pa and 50 Pa, most preferably in the range of between 2 Pa and 25 Pa.

In an embodiment of the method described herein cell viability remains in the range of between 70% to 100% (data not shown).

In another embodiment of the method described herein the cell debris volume as determined using a FlowCam (8000 Analyzer, Fluid Imaging Technologies Inc,. A 10x objective) with the respective flow cell does not exceed 2% i.e. is in the range of 0,01% to 2%.

In an embodiment of the method described herein cell viability remains in the range of between 70% to 100% and the cell debris volume as determined by FlowCam does not exceed 2% i.e. is in the range of 0,01% to 2%.

In a preferred embodiment the shear component (2) is a passive structure and selected from the group consisting of
- a nozzle,
- a filter,
- a strainer,
- a static mixer,
- an inline tube decreasing the diameter of the at least one recirculation loop (3),
- a mesh membrane

In an alternative preferred embodiment the shear component (2) is an active device and selected from the group consisting of a pump, an ultrasonic device and a Completely Stirred Tank Reactor (CSTR).

To a person skilled in the art it is clear that different shear components (2) can be combined.

Exemplary shear devices are rheometers allowing for defined shear rates as well as plate/plate, plate/cone and rotational cylinder devices.

As used herein, the terms "cell agglomeration", "cell aggregate" and "cell oligomer" refer to a plurality of cells in which an association between the cells is caused by cell-cell interaction (e.g., by biologic attachments to one another). Biological attachment may be, for example, through surface proteins, such integrins, immunoglobulins, cadherins, selectins, or other cell adhesion molecules. For example, cells may spontaneously associate in suspension and form cell-cell attachments (e.g., self-assembly), thereby forming aggregates, or cells may be forced to aggregate.. In some embodiments, a cell agglomeration/ aggregate/oligomer may be substantially homogeneous (i.e., mostly containing cells of the same type).

In some embodiments, a cell agglomeration/ aggregate/oligomer may be heterogeneous, (i.e., containing cells of more than one type).

The average diameter of each cell agglomerations is in the range of between >30 µm and 800µm - i.e. in the range of between 30.1 µm and 800 µm - whereas single cells and cell oligomers i.e. cell duplets or triplets have diameters in the range of 8-30 µm. To a skilled person it is clear that depending on the cell type the diameter range of 8µm-30µm for cell oligomers i.e. cell duplets or triplets could be larger. Depending on the application and the cell type it is not necessary that small cell agglomerations at the lower end of the diameter range e.g. of around 30µm are further dissociated, but that the overall diameter range of all cell aggregates is more uniform.

Preferably the average diameter of cell agglomerations is in the range of between > 30 µm and 300µm e.g. 50µm to 300µm. This can be measured via microscopy e.g. using a FlowCam.

As used herein, the terms "passage" and "passaging" refer to the process of sub-culturing adherent cells, in which cell adhesion is disrupted and the cell density (number of cells per unit volume or area) is reduced by addition of fresh medium. Passaging is a technique that may be used to expand the number of cells in a culture and/or prolong the life of a cell line or the cells in culture.

As used herein, the term "expansion" refers to the proliferation of a cell, with or without differentiation, and may include no passaging, one passage or more than one passage and/or serial passages.

As used herein, the terms "dissociate" and "dissociation" refer to a process of separating aggregated cells from one another. For example, during dissociation, the cell-cell interaction between cells and between cells and extracellular matrix in the aggregate may be disrupted, thereby breaking apart the cells in the aggregate.

As used herein, the terms "dissociated" and "dissociated aggregate" refer to single cells, or cell aggregates or clusters that are smaller than the original cell aggregates (i.e., smaller than a pre-dissociation aggregate). For example, a dissociated aggregate may comprise about 50% or less surface area, volume, or diameter relative to a pre-dissociation cell aggregate.

As used herein, the term "dissociation reagent" refers to a solution comprising one or more agents that separate cells from one another, such as, for example, enzyme(s) and/or chelating agent(s). For example, a dissociation reagent may break the bonds between cells and/or between extracellular matrix proteins and cells, thereby disrupting the aggregation of cells in suspension. For example, a dissociation reagent may cause sequestration of a molecule to weaken or break bond formation between cell adhesion proteins (e.g. chelation to disrupt calcium dependent adhesion molecules), it may cause cleavage of proteins (e.g., serine proteases such as trypsin, collagenases, dispase, or papain) and/or other extracellular matrix components (e.g., hyaluronidase) to which cell surface molecules bind or within which cells becomes entrapped, and/or cleave such binding molecules from the cell surface (e.g., cleavage of integrins by serine proteases).

As used herein the term "recirculation loop" refers to a device which allows cycling of the cells in suspension i.e. if cycled twice on average each cell of a cell suspension should pass a given point of the device twice . In some embodiments the recirculation loop does not comprise a bioreactor.

In a preferred embodiment of the method of dissociating cells described herein the method further also comprising a chemical dissociation step and/or increasing the temperature e.g. via an inline heat exchanger for heating the dissociation media and/or solution with dissociating cells. Preferably the chemical dissociation is carried out after or while the shear component (2) exerts the shear stress in the range of between 2 Pa and 500 Pa on the cells to be dissociated thereby reducing the time cells must spend in a single cell state, which decreases their viability.

In a preferred embodiment of the method of dissociating cells described herein the recirculation ratio is in the range between 3-10 (cf. example 4)

The recycle ratio is the volume of cell suspension returned to the shear device entrance divided by the volume leaving the system, i.e. the volume processed by (2) and leaving the system (5).

In a preferred embodiment of the method of dissociating cells described herein the method is continuous or semi-continuous. If the method is continuous and "one way" this ensures that each cell (aggregate) is only stressed once.

In a preferred embodiment the method of dissociating cells described herein the shear component is a nozzle with a diameter in the range of between 0.5mm - 2 mm, preferably 0.7mm -1.5mm.

In a further preferred embodiment the method of dissociating cells described herein the shear component is a nozzle with a diameter of 1.5 mm and the cells to be dissociated pass the nozzle at a flow rate of rate of 19.3 - 62.5 (mL/min) and a velocity of between 0.18 and 0.59 (m/s).

In a further preferred embodiment the method of dissociating cells described herein the shear component is a nozzle with a diameter of 1 mm and the cells to be dissociated pass the nozzle at a flow rate of rate of 41.1 (mL/min) and a velocity of 0.87.

Preferably the cells to be dissociated are chosen from the group consisting of
- Pluripotent stem cells e.g. iPSC cells, human embryonic stem cells, mouse embryonic stem cells
- Chinese Hamster Ovary cells
- Baby Hamster Kidney (BHK) cells
- Differentiated cells, such as gem cells, ectoderm derivatives, endoderm derivatives and mesoderm derivatives.

In a second aspect what is described herein relates to cell dissociation device (1) for dissociating cell agglomerations in the range of between >30 µm to 300 µm into single cells or cell oligomers in the range of 8-30µm comprising
- at least one shear component (2) exerting a maximal shear in the range of between 2 Pa and 500 Pa
- at least one recirculation loop (3) with a volume in the range of between 200 ml and 1000 ml
- at least one recirculation component (4)
- at least one inlet (5)
- and at least one outlet (6).

The combination of recirculation loop and the defined shear component reduces the variability of shear stress encountered by cells and still ensured a defined dissociation at an adequate shear value required to provide a consistent cell quality. Hence via using the device and the method described herein cells suitable for cell therapy applications can be provided on a large scale.

The at least one recirculation loop can be made of single use tubing and preferably has a tubing length in the range of between 0.3 m and 10 m with inner tubing diameters between 3mm and 3 cm. It can comprise a bubble trap and/or a single use reactor

In a preferred embodiment the recirculation loop (3) is has a small volume of no more than the volume supplied via inlet (5) more preferred less than 20%, better less than 10% of the volume supplied via inlet (5) thereby further reducing the variability of the total shear stress encountered by each cell in a cell suspension. For example if the cells were cultured in a total culture volume of 1,3 liter a maximum recirculation volume of 200 mL would be used. In a preferred embodiment of the method of dissociating cells described herein the volume of (cell culture) fluid within the recirculation shear device is smaller than 100 mL.

Preferably the at least one recirculation component (4) is a pump.

The at least one device (1) can further comprise
- at least one a bubble trap
- At least one further pump
- At least one sensor, e.g. such as flow sensor, pressure sensor, temperature sensor,
- At least one additional vessel.

In another preferred embodiment the cell dissociation device comprises at least one cell retention device. This allows for the removal of single cells prior to dissociation as the single cells do not have to be dissociated further. Examples for cell retention devices are cell settlers, sedimentation devices, hydrocline devices, centrifuges and ultrasonic separation devices and filtration devices.

In another preferred embodiment the cell dissociation device comprises at least one additional inlet e.g. to add a dissociation reagent.

Preferably all components of the device (1) are single-use components.

Preferably the device (1) comprises a rheomicroscope as this allows to consistently quantify which shear stress was applied to cell agglomerations.

### EXAMPLES

### Example 1 Determining whether a given and/or envisaged shear component exerts the requires shear part a) PMMA experiments

Examples 1 and 2 were conducted to correlate a given shear stress to a corresponding particle size and vice versa so that a person skilled in the art can determine whether a given device, exerts the required shear stress.

PMMA particles were prepared as described in Villiger 2015 . The experimental setting was as described in example 3 below. Hence based on the publication of Soos et al. 2010 (Langmuir 2010, 26, 1, 10-18) a contracting nozzle was designed and (Custom made, Sausen Maschinenbau GmbH) connected to two serological pipettes (50 mL Costar, Corning Inc.) using a silicon tubing, 7 cm in length/ 4.8 mm in Inner-diameter (FisherScientific)) Moreover a liquid suspension comprising the PMMA particles in demineralized water with Kolliphor^{®} P 188 Geismar (0.01 g/L) and Ex-cell^{®} Antifoam gamma irradiated (0.0045 g/L) was prepared. The nozzle as well as both serological pipettes were placed in a double-walled 5 L bioreactors (bbi-biotech GmbH) connected to a Thermostat (CF41, Julabo) in order to maintain a defined temperature of the liquid suspension. One serological pipette was connected to a syringe pump (Syrdos, HiTec Zang)to adjust the flow rate accurately through the nozzle.

Different nozzles diameters and different flow rates were used according to Table 1 and the PMMA particle dissociation was measured using a FlowCam 8000 Analyzer, Fluid Imaging Technologies Inc,. A 10x objective with the respective flow cell was used. The PMMA particles were diluted to 100.000 particles/mL. The sample volume was 150 µL. Using a calibration curve the PMMA clustersize was correlated to the hydrodynamic stress i.e. the (shear stress).

**Table 1 Tested parameters using PMMA**

| Nozzle ID (mm) | Flow rate (mL/min) | Re nozzle (-) | Velocity (m/s) | Particle size (µm) |
|---|---|---|---|---|
| 1.5 | 19.3 | 392 | 0.18 | 60,26 |
| 1.5 | 35.6 | 724 | 0.34 | 28,51 |
| 1.5 | 62.5 | 1271 | 0.59 | 14,35 |
| 1 | 41.1 | 1254 | 0.87 | 8,89 |
| 1.5 | 147.2 | 2991 | 1.39 | 5,05 |
| 1.5 | 234.0 | 5983 | 2.21 | 2,86 |

### Example 2 Determining whether a given and/or envisaged shear component exerts the requires shear part b) CFD Simulations

In parallel to the PMMA experiments, it was elucidated whether the nozzle geometry could also be simulated via Computational Fluid Dynamics (CFD). In detail, a two-dimensional CFD model was created, exploiting rotational symmetry to describe the physical shear stress inside of a nozzle with defined geometry and corresponding flow field.. The incompressible, laminar flow was predicted by means of steady-state simulations. Similar to Soos et al. (2010) Langmuir 2010, 26, 1, 10-18, (https://doi.org/10.1021/la903982n). The maximum shear stress parts of the aggregates were exposed to the shear during a single nozzle pass. Turbulent origin of shear stress was found to be minor compared to the laminar shear. By varying the nozzle throat diameter between 0.7 to 1.5 mm and the liquid throat velocity in the range of 0.1 to 3.5 m/s different maximal shear stress values were modelled to be exerted to a fluid suspension as summarized in Table 2. In Table 2 the velocity results from the nozzle inner diameter and the flow rate whereas the shear stress results in the CFD simulation depending on the velocity.

**Table 2 Simulated geometry and resulting simulated shear stress**

| Nozzle ID (mm) | Flow rate (mL/min) | Re nozzle (-) | Velocity (m/s) | Stress (Pa) |
|---|---|---|---|---|
| 1.5 | 19.3 | 392 | 0.18 | 2.4 |
| 1.5 | 35.6 | 724 | 0.34 | 6 |
| 1.5 | 62.5 | 1271 | 0.59 | 13.9 |
| 1 | 41.1 | 1254 | 0.87 | 25 |
| 1.5 | 147.2 | 2991 | 1.39 | 50 |
| 1.5 | 234.0 | 5983 | 2.21 | 100 |
| 1 | 216.8 | 6611 | 4.60 | 300 |
| 0.7 | 237.7 | 10354 | 10.29 | 1000 |

Via combining the velocity corresponding to a given shear stress and the particle size corresponding to the same velocity in the PMMA experiment (cf. Example 1 above) it could be determined which shear stress for a given nozzle geometry and a given velocity results in which particle size. Thus for each particle size now the corresponding shear stress was known. Hence e.g. in a different shear stress device if a certain particle size was measured e.g. via PMMA method then the shear stress that had caused the particle size could be deducted. Hence the parallel CFD and PMMA experiments described in Examples 1 and 2 do not have to be repeated again but for a different shear device it is sufficient to use one method to determine which shear stress is expected as the shear stress and the particle size have already been correlated.

### Example 3 Determination of shear stress required for cell dissociation

After conducting the PMMA experiments and CFD simulations as described above in examples 1 and 2, respectively it was determined how much shear was required to dissociate cells. In order to do so a contracting nozzle was custom made by Sausen Maschinenbau GmbH. The nozzle was connected to two serological pipettes (50 mL Costar, Corning Inc.) using a Silicon tubing, 7 cm in length/ 4.8 mm in inner-diameter (FisherScientific) . Moreover a liquid suspension consisting of DMEM/F12 Cell Culture Medium, ThermoFisher Scientific and iPS Cells (0.5 - 1.5×10⁶ Cells/mL) was prepared. The nozzle as well as both serological pipettes were placed in a double-walled 5 L bioreactors (bbi-biotech GmbH) connected to a Thermostat (CF41, Julabo) in order to maintain a defined temperature of the liquid suspension/cell suspension. One serological pipette was connected to a syringe pump (Syrdos, HiTec Zang) to adjust the flow rate accurately through the nozzle. The complete liquid suspension comprising he cells was pumped through the nozzle 200 times as Villiger et al. 2015 (Villiger et al AIChEJ 61-2015) showed in Fig. 4 that around 200 passes are required for PMMA particles to achieve a steady aggregate size.

Different nozzles diameters and different flow rates were tested according to Tab. 3.

**Table 3 Experimental Conditions for Nozzle Shear experiments**

| Nozzle ID (mm) | Flow rate (mL/min) | Re nozzle (-) | Velocity (m/s) | Stress (Pa) |
|---|---|---|---|---|
| 1.5 | 19.3 | 392 | 0.18 | 2.4 |
| 1.5 | 35.6 | 724 | 0.34 | 6 |
| 1.5 | 62.5 | 1271 | 0.59 | 13.9 |
| 1 | 41.1 | 1254 | 0.87 | 25 |
| 1.5 | 147.2 | 2991 | 1.39 | 50 |
| 1.5 | 234.0 | 5983 | 2.21 | 100 |
| 1 | 216.8 | 6611 | 4.60 | 300 |
| 0.7 | 237.7 | 10354 | 10.29 | 1000 |

Cell damage was measured in terms of cell debris Biovolume (%) using a FlowCam (8000 Analyzer, Fluid Imaging Technologies Inc,. A 10x objective) with the respective flow cell. Cells were diluted with PBS buffer to 100.000 cells/mL. The sample volume was 150 µL

The cell debris consisted of cell fragments derived from necrotic cells which in this experiment were predominantly the result of the shear stress and hence cell debris was taken as measure of shear stress. The cell debris biovolume was used to normalize the generated cell debris by dividing the volume of cell debris by the total volume of objects detected with the FlowCam Analyzer assuming all particles and debris had a spherical shape.

The results given in Table 4 showed that at a shear stress above 25 Pa considerably more cell debris appeared.

**Table 4 Cell debris amount in relation to shear stress after 200 nozzle passes**

| | Shear stress in PA | 2,4 | 6 | 14 | 25 | 50 | 100 | 300 | 1000 |
|---|---|---|---|---|---|---|---|---|---|
| Cell debris In % Biovolume | | 0,5 | 0,7 | 0,5 | 0,4 | 0,85 | 1,2 | 1,5 | 2 |

In detail it can be seen that in the range of between 2 and 25 Pa corresponding to a nozzle diameter of 1,5 mm at a flow rate of 19.3 - 62.5 and a velocity of between 0.18 and 0.59 or a nozzle diameter of 1 mm at a flow rate of 41.1 and a velocity of 0.87. the percentage of biovolume representing cell debris substantially remains the same indicating that a shear in this range does not adversely affect the cells. In contrast, if a shear in the range of 30- 1000 Pa is applied the cell debris percentage of the biovolume increases noticeably indicating that this shear adversely affects the cells.

**Table 5 Dissociation in relation to shear stress after 200 nozzle passes**

| Shear stress in Pa | 0 | 2,4 | 6 | 14 | 25 | 50 | 100 | 300 | 1000 |
|---|---|---|---|---|---|---|---|---|---|
| Single cells In % Biovolume | 3 | 10 | 13 | 20 | 18 | 12 | 30 | 40 | 51 |
| Cell aggregates > 30µm in % Biovolume | 78 | 63 | 54 | 36 | 41 | 58 | 20 | 6 | 4 |
| Ratio single cells: cell aggregates > 30µm in % Biovolume | 1:26 | 1:6,3 | 1:4,2 | 1:1,8 | 1:2,3 | 1:4,8 | 1:1,5 | 1:0,15 | 1:0,08 |

Table 5 demonstrates that in order to dissociate nearly all cell agglomerations into single cells at 200 nozzle passes shear stress values of 50 Pa and above were necessary (Single cells were defined as cells without attachment to other cells). However, as Table 4 showed that shear stress values of around 50 Pa already considerably increase the cell debris volume it was determined in Example 4 how many nozzle passes at a shear below 50 Pa were necessary to reach an acceptable ratio of single cells to : cell aggregates > 30µm.

### Example 4 Determining the optimal number of nozzle passes

Apart from determining the shear necessary to dissociate cells agglomerations without damaging the cells also the optimal recycle ratio was assessed. The experimental set up was the same as described for example 3 except that the flow rate was not varied but kept constant at 62.5 mL/min to achieve a shear of 13.9 Pa. The nozzle with the diameter of 1.5 mm was used and the cells were passed several times (cf. Table 6) through the nozzle. Aggregate size was measured by a FlowCam [FlowCam 8000Analyzer, Fluid Imaging Technologies Inc] . A 10x objective with the respective flow cell was used. Cells were diluted with PBS buffer to 100.000 cells/mL. The sample volume was 150 µL. Single cells were defined as cells without attachment to other cells.

**Table 6**

| Nozzle passes | 0 | 3 | 10 | 50 | 100 | 200 |
|---|---|---|---|---|---|---|
| Single cells In % Biovolume | 8 | 17 | 20 | 20 | 17 | 23 |
| Cell aggregates > 30µm in % Biovolume In % Biovolume | 72 | 46 | 39 | 40 | 49 | 36 |
| Duration of experiment | - | 2min | 6min | 32 min | 64 min | 128 min |

It can be seen that increasing number of nozzle passes promoted dissociation with between 3-10 nozzle passes being enough for most distinct effects.

Therefore, using the method and the device as described herein with 3-10 nozzle passes allows for using a shear component exerting less stress by reducing the time the cells are exposed to the shear to a comparatively short time while still achieving the required dissociation of cell agglomerations of >30 µm to 300 µm into single cells or cell oligomers in the range of 8µm to 30µm.

## Claims

1. Method for dissociating cell agglomerations in the range of between >30 µm to 300 µm after harvest into single cells or cell oligomers in the range of 8-30 µm comprising the steps of
• providing cell agglomerations in the range of between >30 µm to 300 µm
• passing said cell agglomerations in the range of between >30 µm to 300 µm through a recirculation loop
• bringing said cell agglomerations in the range of between >30 µm to 300 µm into contact with a shear component exerting a maximal shear in the range of between 2 Pa and 500 Pa
thereby dissociating the cell agglomerations in the range of between >30 µm to 300 µm into single cells or cell oligomers in the range of 8-30 µm.

2. Cell dissociation device (1) for dissociating cell agglomerations in the range of between >30 µm to 300 µm into single cells or cell oligomers in the range of 8-30µm comprising
• at least one shear component (2) exerting a maximal shear in the range of between 2 Pa and 500 Pa
• at least one recirculation loop (3) with a volume in the range of between 200 ml and 1000 ml
• at least one recirculation component (4)
• at least one inlet (5)
• and at least one outlet (6).

3. Method according to claim 1 or device according to claim 2 wherein the maximal shear exerted by the least one shear component (2) is in range of between 2 Pa and 25 Pa.

4. Method according to claim 1, wherein the cell agglomerations have an average diameter of 100-200µm.

5. Method according to claim 1, wherein the recirculation ratio is in the range between 3-10.

6. Method according to claim 1 or device according to claim 2 wherein the shear component (2) is a nozzle with a diameter in the range of between 0.5mm - 2 mm.

7. Device according to claim 2 wherein the cell dissociation device (1) comprises at least one cell retention device.
